# EUROPEAN PATENT APPLICATION

(11) **EP 3 181 181 A1**
(43) Date of publication of application: **21.06.2017**
(21) Application number: 15825150.4
(22) Date of filing: 23.04.2015
(51) Int. Cl.: A61M 37/00, A61K 9/16, A61K 31/07, A61K 47/30

(54) **MICRONEEDLE CONTAINING RETINOL OR RETINOL DERIVATIVE**

(30) Priority: 24.07.2014 KR 20140094220
(71) Applicant: LG Household & Health Care Ltd., Seoul 110-783 (KR)
(72) Inventor: SHIM, Woo-Sun, Daejeon 305-343 (KR); KIM, Dong-Chan, Daejeon 305-343 (KR); HWANG, Young-Min, Daejeon 305-343 (KR); LEE, Sun-Hwa, Daejeon 305-343 (KR); KANG, Nae-Gyu, Daejeon 305-343 (KR); PARK, Sun-Gyoo, Daejeon 305-343 (KR); LEE, Cheon-Koo, Daejeon 305-343 (KR)
(74) Representative: Goddar, Heinz J.
(86) International application number: PCT/KR2015/004070
(87) International publication number: WO 2016/013755

(57) **Abstract**

The present disclosure relates to a system for administering retinol into skin that ensures stability of retinol, reduces skin irritation of retinol, and continues to exert effects, wherein the system is a microneedle including microparticles containing retinol or retinol derivatives. The present disclosure further provides a method for manufacturing a retinol administration system that reduces skin irritation, exerts effects of retinol for a long time, and ensures stability of retinol very unstable to light, wet and temperature. The present disclosure further provides a method for administering retinol into skin using the microneedle.

## Description

### TECHNICAL FIELD

The present disclosure relates to a system for administering retinol to skin, in particular, a microneedle containing retinol, that reduces side effects such as skin erythema, improves stability of retinol and has an intended effect for a long time, and a method for manufacturing the same.

The present application claims priority to Korean Patent Application No. 10-2014-0094220 filed in the Republic of Korea on July 24, 2014, the disclosure of which is incorporated herein by reference.

### BACKGROUND ART

Retinol is a type of vitamin A, and plays an important role in maintaining intrinsic function of epidermal cells of skin. Specifically, retinol is known to promote cell differentiation by inducing DNA in cell nucleus found in skin cells to express RNA, and to promote biosynthesis of scleroprotein present in fibrous solid form between animal cells such as elastin composed of collagen and elastic fibers, thereby having efficacy on wrinkle reduction and skin elasticity improvement.

For this reason, development started to develop retinol long before for cosmetic applications to eliminate wrinkles or prevent skin aging, and in the late 1990s, cosmetics companies in the world launched cosmetic products containing retinol successively, triggering intense market dominance competition between companies.

However, in the case of retinol products, many problems were found. Specifically, retinol has superior effects, while retinol is easily destroyed by light, temperature and oxygen, so when retinol is used as a raw material for cosmetics, stability that allows retinol to get into skin is the most important. If a retinol component is oxidized and deteriorated by an external factor, it does not work and even causes adverse effects. To solve the problem, expensive special containers are used, the condition of use is limited to places where there is no light, or it is recommended that cosmetics be used up within three months once the cosmetics are opened, but there are many accompanying inconveniences.

To address the stability-related issue, retinyl acetate and retinyl palmitate with superior stability may replace retinol. However, because retinol derivatives fail to penetrate deep into skin and synthesize protein like retinol, stability is high but effects are relatively low.

On the other hand, retinol often causes skin irritation such as erythema when it is absorbed into skin.

### DISCLOSURE

### Technical Problem

Therefore, the problem to be solved by the present disclosure is to provide a retinol administration system that reduces skin irritation of retinol, ensures stability of retinol in the administration system, and exerts effects of retinol for a long time, and a method for manufacturing the system and a method for administering retinol using the system.

### Technical Solution

To solve the problem, the present disclosure provides a microneedle including microparticles containing retinol or retinol derivatives, and more preferably, a material that forms the microneedle is dissolved in skin, and the microneedle is thus dissolved or destructed when the microneedle is applied to skin, so microparticles included in the microneedle are rapidly released into skin, and as the microparticles included inside include a sustained release polymer, retinol or its derivatives are released in skin in small amounts at a time.

The inventors have studied various administration systems, and any system was not easy to solve all the many problems of retinol mentioned above. After a lot of efforts, the inventors made a surprising invention by impregnating microparticles into a soluble microneedle in skin wherein the microparticles are made of a sustained release polymer and contain a retinol component, thereby solving the stability, skin irritation, and effect sustainability issues of retinol at one time. When the microparticles containing the retinol component encapsulated therein are impregnated into the soluble microneedle, which is applied to skin, then the retinol component penetrates into skin without pain caused by the microneedle, and as the microneedle is dissolved by moisture in skin, the microparticles containing the retinol component encapsulated therein are delivered into skin. The retinol component is slowly released from the microparticles delivered into skin, thereby reducing skin irritation resulting from retinol and maximizing retinol effects.

To achieve the object, the microneedle should be soluble in skin, and to form the soluble microneedle, a water-soluble polymer may be used, such as hyaluronic acid, sodium carboxymethyl cellulose (Na-CMC), vinyl pyrrolidone-vinyl acetate copolymer, poly vinyl alcohol, and poly vinyl pyrrolidone; saccharide such as xylose, sucrose, maltose, lactose, and trehalose; or mixtures thereof. Particularly, when considering the force of the microneedle to penetrate skin and the rate of dissolution in skin together, a mixture of oligo-hyaluronic acid, sodium carboxymethyl cellulose (Na-CMC), and saccharide (more preferably, trehalose) is more preferable, and an addition of glycerine described below to the mixture is much more preferable.

Preferably, in addition to the microparticles containing the retinol component and the above-mentioned ingredients that form the microneedle, the microneedle according to the present disclosure may further include a plasticizer, a surfactant, a preservative, and an anti-inflammatory agent.

The plasticizer may include polyols such as, for example, ethylene glycol, propylene glycol, dipropylene glycole, butylene glycol, and glycerine, singly or in combination.

In the present disclosure, the material that forms the microparticles together with the retinol component should be a material that does not dissolve or disperse and disappear in the manufacture of the microneedle and thus can hold the retinol component in the microparticles, while at the same time, achieving the sustained release of the retinol component in skin.

The material that forms the microparticles includes a sustained release polymer, and the sustained release polymer includes, but is not limited to, biodegradable polymer such as poly(lactide), poly(glycolide), poly(lactide-co-glycolide), polyanhydride, polyorthoester, polyetherester, polycaprolactone, mono-methoxy polyethyleneglycol-polycaprolactone (MPEG-PCL), polyesteramide, poly(butyric acid), poly(valeric acid), polyurethane, or copolymers thereof; and non-biodegradable polymer such as polyacrylate, ethylene-vinylacetate copolymer, acryl substituted cellulose acetate, non-degradable polyurethane, polystyrene, polyvinyl chloride, polyvinyl fluoride, poly(vinyl imidazole), chlorosulphonate polyolefins, polyethylene oxide or copolymers thereof, singly or in combination.

When considering stability of the retinol component and release in skin together, the sustained release polymer is much more preferably a mixture of at least one of poly(lactide), poly(glycolide) and poly(lactide-co-glycolide), and mono-methoxy polyethyleneglycol-polycaprolactone (MPEG-PCL).

The microparticles may be of a matrix type and a reservoir type so long as the objective of the present disclosure is attained.

The microparticles available for the present disclosure may be manufactured by a variety of methods well known in the field to which the present disclosure belongs. For example, the microparticles available for the present disclosure may be manufactured using a solvent exchange method, a solvent evaporation method, a membrane dialysis method, and a spray drying method. For example, methods described in Journal of Controlled Release 70 (2001) 1-20 and International Journal of PharmTech Research, 3(2011) 1242-1254 may be used. Preferably, the microparticles may be manufactured by a general emulsification and solvent evaporation method.

Preferably, the microparticles according to the present disclosure has a diameter of from 0.01 to 10 µm. When the particle size exceeds 10 µm, the needle strength reduces, making skin penetration difficult, when the microparticles are impregnated into the microneedle. The diameter of the microparticles according to the present disclosure is measured by a laser light scattering (LLS) method, for example, using Malvern Zetasizer 2000™.

Preferably, the microparticles of the present disclosure contain retinol or retinol derivatives in an amount of 0.01 to 10 weight%, more preferably 0.1 to 5 weight%, per the total weight of the microparticles. Furthermore, the microneedle of the present disclosure preferably contain the microparticles in an amount of 0.05 to 10 weight%, more preferably 0.1 to 5 weight%, per the total weight of the microneedle.

The retinol derivatives available for the present disclosure include retinyl acetate, retinyl palmitate, retinol or mixtures thereof, but the present disclosure is not limited to a particular type of retinol derivatives.

Furthermore, the present disclosure provides a microneedle patch system for administering (delivering) a retinol component, including the above-mentioned microneedle attached thereto.

The present disclosure further provides a method for manufacturing a microneedle containing retinol, including (S1) manufacturing microparticles containing retinol or retinol derivatives using the above-mentioned sustained release polymer, and (S2) manufacturing a microneedle containing the microparticles using a soluble material in skin, thereby reducing irritation of the retinol component, ensuring stability of the retinol component, and continuing to exert effects.

The present disclosure further provides a method for administering retinol into skin using the microneedle according to the present disclosure, thereby reducing skin irritation of retinol and achieving sustained release of retinol.

### Advantageous Effects

The present disclosure provides a microneedle for administering retinol into skin that ensures stability of retinol, reduces skin irritation of retinol, and continues to exert effects. The present disclosure further provides a method for manufacturing a retinol administration system that reduces skin irritation, exerts effects of retinol for a long time, and ensures stability of retinol very unstable to light, wet and temperature. The present disclosure further provides a method for administering retinol into skin using the microneedle.

### BRIEF DESCRIPTION OF THE DRAWINGS

The accompanying drawings illustrate preferred embodiments of the present disclosure and together with the foregoing disclosure, serves to provide further understanding of the technical spirit of the present disclosure, and thus, the present disclosure is not construed as being limited to the drawing.

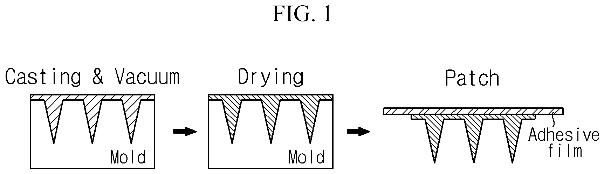

FIG. 1 is a diagram showing one of many methods for manufacturing a microneedle according to the present disclosure. The soluble microneedle may be manufactured by a solution casting method, whereby a solution is casted into a mold and mold cavities are filled with the solution by vacuum and centrifuge, followed by drying. A material that forms the microneedle structure includes general synthetic and natural water-soluble polymers.
FIG. 2 shows a Franz diffusion cell for evaluating retinol release behaviors of a microneedle according to the present disclosure.
FIG. 3 is a graph showing evaluation results of retinol release from a microneedle, as evaluated using a Franz diffusion cell having on pig skin set thereon.
FIG. 4 is photographic results showing the extent of skin irritation appearing after application to skin, for a microneedle containing retinol itself (added in emulsion form), a microneedle containing retinol microparticles according to the present disclosure, and general cream formulation containing retinol microparticles.
FIG. 5 is graph results showing the skin irritation index appearing after repeated use on skin, for a microneedle containing retinol itself (added in emulsion form), a microneedle containing retinol microparticles according to the present disclosure, and general cream formulation containing retinol microparticles.
FIG. 6 is test results showing the extent of wrinkle improvement appearing after long-term use of a microneedle containing retinol microparticles according to the present disclosure and general cream formulation containing retinol microparticles.

### BEST MODE FOR CARRYING OUT THE INVENTION

Hereinafter, the present disclosure is described in detail based on embodiments to help the understanding of the present disclosure. However, the embodiments according to the present disclosure may be modified in many different forms, and the scope of the present disclosure should not be construed as being limited to the following embodiments. The embodiments of the present disclosure are provided to explain the present disclosure to those skill in the art fully and completely.

### <Manufacture of retinol microparticles>

10g poly lactic acid (PLA) and 10g mono-methoxy poly ethylene glycole - poly carprolactone (MPEG-PCL) diblock copolymer was dissolved in 100g methylene chloride, and then mixed with 5g retinol 50C (BASF, retinol 50%) to prepare a polymer-retinol solution. 2.5g poly vinyl alcohol (PVA) was dissolved in 500mL purified water to prepare a PVA aqueous solution. The polymer-retinol solution was slowly added while shaking 500mL PVA aqueous solution at 700rpm, to prepare an oil in water emulsion solution. Shaking was performed at 700rpm for 24 hours to evaporate an organic solvent methylene chloride, yielding stable retinol microparticles. Using a rotary evaporator, concentration was performed by completely removing the remaining methylene chloride while evaporating water together with the organic solvent so that the retinol content is 3% in total.

As a result of analysis by liquid chromatography, the retinol content was found to be 2.9%, and as a result of analysis using a particle size analyzer (Malvern Zetasizer 2000)™, the average size of the microparticles was 450nm.

### <Manufacture of a microneedle containing retinol or retinol microparticles>

As in the following table 1, a microneedle containing retinol (added in emulsion form) or retinol microparticles was manufactured.

**[Table 1]**

| | R MN | R-MP MN |
|---|---|---|
| oligo-HA | 6 | 6 |
| Na-CMC | 6 | 6 |
| Trehalose | 10 | 10 |
| Glycerin | 5 | 5 |
| HCO-40 | 0.2 | 0.2 |
| Retinol 50C | 0.04 | - |
| Retinol microparticle (3%) | - | 0.7 |
| water | To 100 | To 100 |

Specifically, a soluble microneedle containing impregnated retinol(R MN) was manufactured as follows. Oligo-HA (Hyaluronic acid), Na-CMC (Sodium carboxymethyl cellulose) and Trehalose was dissolved in purified water, and Glycerin, HCO-40 and retinol 50C (BASF, retinol 50%, Tween 20 50%) was added thereto, to prepare a retinol W/O emulsion solution. The prepared retinol emulsion solution was casted into a silicon microneedle mold, and centrifugation was performed at 3000rpm for 10 minutes to fill mold cavities with the solution. After filling with the solution, drying was performed in a dry oven (70°C) for 3 hours, and the microneedle was separated from the silicone mold using an adhesive film.

Specifically, a soluble microneedle containing impregnated retinol microparticles (R-MN) was manufactured as follows. Oligo-HA (Hyaluronic acid), Na-CMC (Sodium carboxymethyl cellulose) and Trehalose was dissolved in purified water, and glycerin, HCO-40 and retinol microparticles (retinol 3%) was added thereto, to prepared a solution. The prepared solution was casted into a silicone microneedle mold, and centrifugation was performed at 3000rpm for 10 minutes to fill mold cavities with the solution. After filling with the solution, drying was performed in a dry oven (70°C) for 3 hours, and the microneedle was separated from the silicone mold using an adhesive film.

### <Retinol release behaviors>

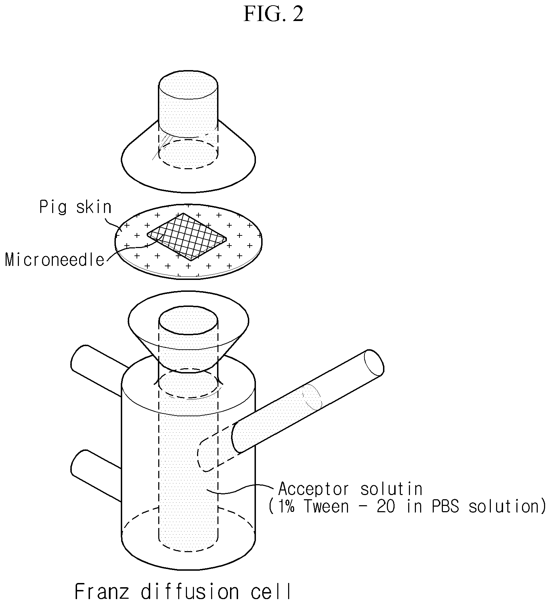

The retinol release from the microneedle manufactured as above was evaluated using a Franz diffusion cell having pig skin set thereon (see FIG. 2). For an acceptor solution, a PBS solution containing 1 wt% Tween 20 was used.

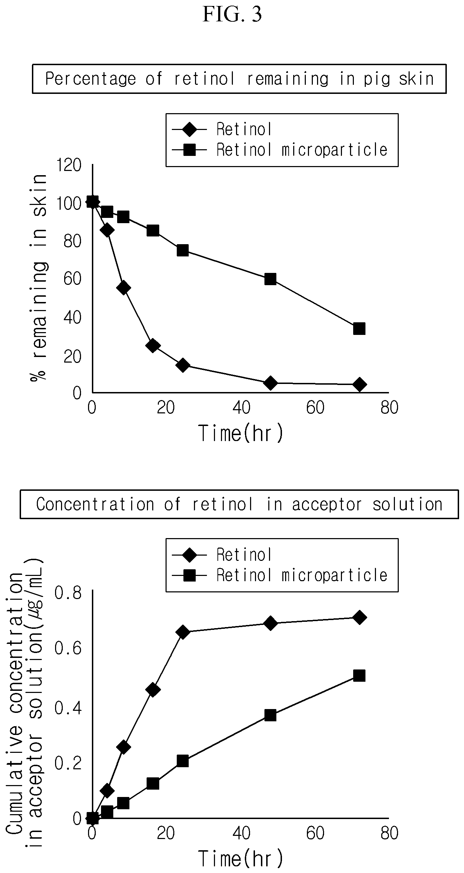

That is, using a Franz diffusion cell, the retinol content in pig skin tissues and acceptor solution over time was measured using liquid chromatography. The microneedle containing impregnated retinol (emulsion) or retinol microparticles was attached to the pig skin to allow the microneedle to penetrate into and dissolve in the pig skin (adhesion time: 2 hours, temperature: 32°C) and then the microneedle was removed. The pig skin into which retinol was absorbed by the microneedle was put in the franz diffusion cell, and release behaviors of retinol from the pig skin to the acceptor solution over time were observed. The results are shown in FIG. 3.

As shown in FIG. 3, for the pig skin into which retinol was penetrated by the microneedle containing impregnated retinol (emulsion), the retinol content in pig skin reduced rapidly over time, and the retinol content in acceptor solution increased quickly, while for the pig skin into which retinol was penetrated by the microneedle containing impregnated retinol microparticles, the retinol content in pig skin was found to reduce slowly, and increase slowly in the acceptor solution. This is because retinol is slowly released from the retinol-microparticles penetrated into the pig skin.

### <Skin irritation test>

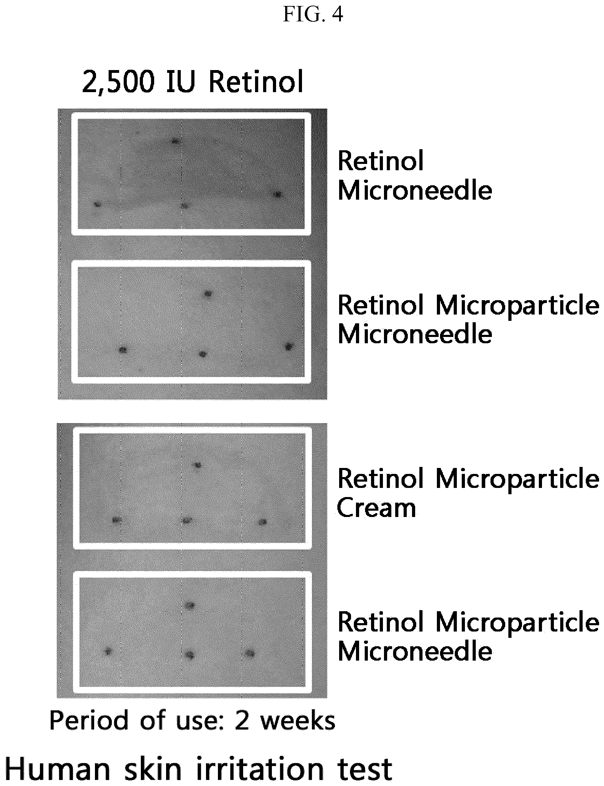

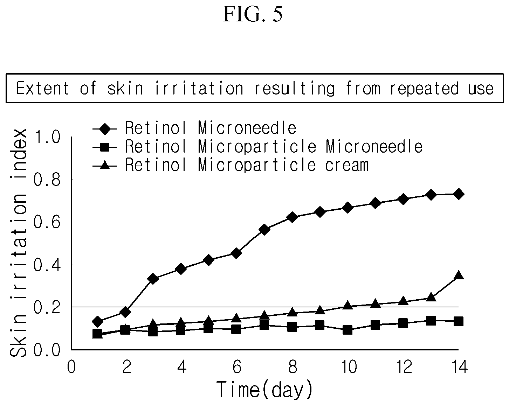

A skin irritation test was conducted by applying each of the microneedle containing retinol; the microneeldle containing impregnated retinol microparticles; and the cream containing retinol microparticles to the inner side of an arm daily for 2 weeks. After adhering the microneedle for 1 hour at a time and applying the cream (N=20, retinol content: 2500IU), the extent of skin irritation was observed. The results are shown in FIGS. 4 and 5.

In the case of the microneedle containing impregnated retinol, severe irritation accompanied by erythema and swelling appeared, while in the case of the microneedle containing impregnated retinol microparticles, skin irritation was as minor as general functional cosmetic level (skin irritation index: about 0.1, and skin irritation index of 0.2 or more is determined as irritated skin, and in the case of functional cosmetics, skin irritation was usually at the level of 0.05∼0.1), and lower skin irritation than the cream containing retinol microparticles was exhibited.

The lower skin irritation found in the microneedle containing impregnated retinol microparticles than the microneedle containing impregnated retinol is because retinol is slowly released from microparticles after retinol penetrates into skin, and it can be inferred from Franz diffusion cell test results. Rather, the present disclosure is not limited to this theoretical mechanism.

Furthermore, it is determined that less skin irritation found in the microneedle containing impregnated retinol microparticles than the cream containing impregnated retinol microparticles is because in practical use, the microneedle is removed after a single use (1 hour), while the cream is applied for a long time, so the time during which the skin touches retinol is short. Rather, the present disclosure is not limited to this theoretical mechanism.

### <Wrinkle improvement effect>

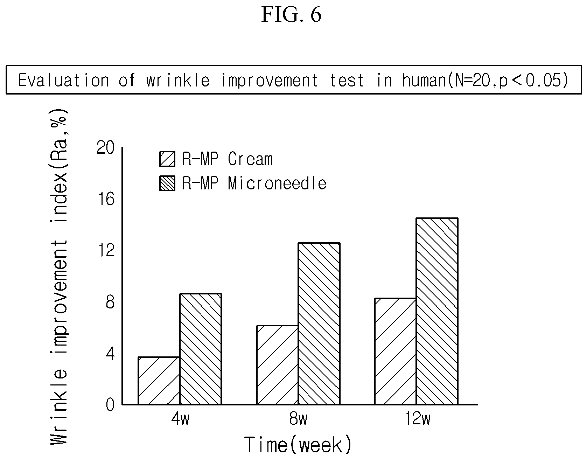

The extent of wrinkle improvement was evaluated after treating wrinkles around eyes with the cream containing retinol microparticles and the microneeedle containing retinol microparticles daily for 12 weeks. The extent of wrinkle improvement was determined through silicone replica and a wrinkle image analysis method (N=20). The results are shown in FIG. 6.

The microneedle containing retinol microparticles according to the present disclosure showed a wrinkle improvement effect at least twice higher than the cream formulation, and it was seen that drug penetration into skin is improved by the microneedle, and the penetrated retinol is slowly released by the microparticles, achieving low skin irritation and high effects.

In the case of the microneedle containing retinol, skin irritation was high, making it difficult to exert a wrinkle improvement effect on humans, and when a retinol emulsion is included in a general cream, retinol in the cream was unstable, so applying general retinol was insignificant in terms of efficacy and effects. Accordingly, a cream containing retinol microparticles was used as control.

## Claims

1. A microneedle comprising microparticles containing retinol or retinol derivatives.

2. The microneedle according to claim 1, wherein a material that forms the microneedle is dissolved in skin, and the microparticles comprise a sustained release polymer.

3. The microneedle according to claim 2, wherein the material that forms the microneedle is hyaluronic acid, sodium carboxymethyl cellulose (Na-CMC), vinylpyrrolidone-vinylacetate copolymer, poly vinyl alcohol, poly vinyl pyrrolidone, saccharides, or mixtures thereof.

4. The microneedle according to claim 3, wherein the microneedle further comprises a plasticizer besides the material that forms the microneedle.

5. The microneedle according to claim 2, wherein the sustained release polymer is poly(lactide), poly(glycolide), poly(lactide-co-glycolide), polyanhydride, polyorthoester, polyetherester, polycaprolactone, mono-methoxy polyethyleneglycol-polycaprolactone (MPEG-PCL), polyesteramide, poly(butyric acid), poly(valeric acid), polyurethane, polyacrylate, ethylene-vinylacetate copolymer, acryl substituted cellulose acetate, non-degradable polyurethane, polystyrene, polyvinyl chloride, polyvinyl fluoride, poly(vinyl imidazole), chlorosulphonate polyolefins, polyethylene oxide, or copolymers or mixtures thereof.

6. The microneedle according to claim 5, wherein the sustained release polymer is a mixture of at least one of poly(lactide), poly(glycolide) and poly(lactide-co-glycolide), and mono-methoxy polyethyleneglycol-polycaprolactone (MPEG-PCL).

7. The microneedle according to claim 1, wherein the microparticles are of a matrix type or a reservoir type.

8. The microneedle according to claim 1, wherein the microparticles have a diameter of from 0.01 to 10 µm.

9. The microneedle according to claim 1, wherein the retinol derivatives are retinyl acetate, retinyl palmitate, retinol, or mixtures thereof.

10. A method for manufacturing a microneedle containing retinol, comprising:
(S1) manufacturing microparticles containing retinol or retinol derivatives using a sustained release polymer; and
(S2) manufacturing a microneedle containing the microparticles using a soluble material in skin.

11. The manufacturing method according to claim 10, wherein the sustained release polymer is poly(lactide), poly(glycolide), poly(lactide-co-glycolide), polyanhydride, polyorthoester, polyetherester, polycaprolactone, mono-methoxy polyethyleneglycol-polycaprolactone (MPEG-PCL), polyesteramide, poly(butyric acid), poly(valeric acid), polyurethane, polyacrylate, ethylene-vinylacetate copolymer, acryl substituted cellulose acetate, non-degradable polyurethane, polystyrene, polyvinyl chloride, polyvinyl fluoride, poly(vinyl imidazole), chlorosulphonate polyolefins, polyethylene oxide, or copolymers or mixtures thereof.

12. The manufacturing method according to claim 10, wherein the soluble material in skin is hyaluronic acid, sodium carboxymethyl cellulose (Na-CMC), vinylpyrrolidone-vinylacetate copolymer, poly vinyl alcohol, poly vinyl pyrrolidone, saccharides, or mixtures thereof.

13. A method for administering retinol into skin using the microneedle according to any of claims 1 through 9 to reduce skin irritation of retinol and achieve sustained release of retinol.
